# EUROPEAN PATENT SPECIFICATION

(11) **EP 3 938 025 B1**
(45) Date of publication and mention of the grant of the patent: **09.08.2023**
(21) Application number: 20716187.8
(22) Date of filing: 10.03.2020
(51) Int. Cl.: A61N 1/04, A61N 1/36, A61B 5/389

(54) **INTEGRATED DEVICE FOR ELECTROMYOGRAPHY AND ELECTROTHERAPY**
INTEGRIERTES GERÄT FÜR ELEKTROMYOGRAPHIE UND ELEKTROTHERAPIE
DISPOSITIF INTÉGRÉ POUR L'ÉLECTROMYOGRAPHIE ET L'ÉLECTROTHÉRAPIE

(30) Priority: 11.03.2019 IT 201900003467
(43) Date of publication of application: 19.01.2022
(73) Proprietor: Nelson & Company S.r.l.s., 00040 Rocca di Papa (RM) (IT)
(72) Inventor: BRANDIMARTE, Bruno, 00048 Nettuno RM (IT); DI TOMASSI, Antonello, 00040 Rocca di Papa RM (IT)
(74) Representative: Manna, Sara
(86) International application number: PCT/IB2020/052049
(87) International publication number: WO 2020/183356

(56) References cited:
- EP-A1- 3 287 871
- WO-A1-2004/067085
- KR-B1- 100 550 673
- US-A1- 2014 148 872
- US-A1- 2015 005 840
- US-A1- 2016 045 746
- US-A1- 2017 049 352
- US-A1- 2017 368 297
- US-A1- 2018 153 477
- HAN GUI ET AL: "Latest Progresses in Developing Wearable Monitoring and Therapy Systems for Managing Chronic Diseases", ARXIV.ORG, CORNELL UNIVERSITY LIBRARY, 201 OLIN LIBRARY CORNELL UNIVERSITY ITHACA, NY 14853, 6 February 2018 (2018-02-06), XP081215302,

## Description

### Technical field of the invention

The present invention relates to an integrated device, configured to detect electromyographic parameters indicative of the functional state of a patient's muscle and deliver an electrical stimulation or an electro-analgesic signal at the same muscle, according to the electromyographic parameters detected.

### Background

To assess the health state of the nerves and muscles, a diagnostic examination named surface electromyography (EMG) is commonly used. This examination investigates the functionality of muscles and nerves by measuring basal electrical potentials being formed at the insertion points of the muscle during its voluntary contraction.

The measurement of potentials is performed by applying electrodes, in particular surface electrodes.

Devices traditionally used for surface electromyographic investigation are surface electromyographs. These devices include detection electrodes connected to a control unit that implements a software for processing the electrical data detected. Using electromyography, it is possible to detect an altered functional state with reference to one or more muscles or muscle groups, for example muscle weakness, spasms, paralysis or pain, which can be consequent to a trauma or disease.

To recover proper muscle function, electrical stimulation therapy of the affected body district is often prescribed. Alternatively, or additionally, electroanalgesia therapy may be provided to temporarily soothe the painful state. These therapies involve the use of an additional device, or an electrotherapy apparatus.

Generally, electrotherapies involve the use of electrodes for the delivery of electrical signals, connected to a control unit implementing a software for the control and regulation of the parameters of the aforementioned signals.

Therapies consist in the delivery to the affected muscles of electrical impulses, and/or more generally sinusoidal signals - which are less annoying and more effective and tolerable for the patient - according to intensity, duration and frequency variable depending on the patient's functional state, on the stage of advancement of therapies and on the desired effect.

EP3287871 A1 discloses a method performed by a wearable device to provide a feedback electrical signal to stimulate muscles of the body part of a user. The method provides to detect a user motion; determine, based on the detected user motion, a body part to which a feedback signal is to be transmitted; generate the feedback signal; and outputting the feedback signal.

US2018153477A1 discloses systems, devices, and methods for detecting stroke in a patient. A system is disclosed which comprises a sensor circuit for sensing in a patient a first physiological signal and a second physiological signal or a functional signal. A stroke risk circuit establishes a physiological trend from at least the first physiological signal over time, and generates a stroke risk indicator using the physiological trend and the second physiological or functional signal. The system includes an output unit that outputs the stroke risk indicator to a user or a process.

The article "Latest Progresses in Developing Wearable Monitoring and Therapy Systems for Managing Chronic Diseases" by Han Gui and Jing Liu, dated 6 February 2018, discloses developments in the field of wearable devices, with particular focus on specific interpretations of therapy for chronic diseases including movement disability after neurologic injuries.

A summary on the key enabling technologies allowing for the implementation of wearable monitoring and therapy devices is given, such as miniaturization of electronic circuits, data analysis techniques, telecommunication strategy and physical therapy.

US2017368297A1 discloses methods and apparatuses for improving sleep by transdermal electrical stimulation (TES) of the subject's head (e.g., temple/forehead region) and/or neck with an TES waveform adapted to improve sleep, including reducing sleep onset (falling to sleep) more quickly and/or lengthening the duration of sleep. TES waveform(s) particularly well suited to enhancing sleep are also described herein.

US2017/049352A1 discloses wearable devices including electrical contacts to detect voltages or other biosignals when mounted to the skin of a wearer. The impedance between a pair of the electrical contacts is detected and the device operated based on the detected impedance. The device detects an electrocardiogram or other biopotentials using the electrical contacts if the detected impedance falls below a specified threshold. The device indicates that the detected impedance is below the specified threshold, e.g., such that a wearer could contact one of the electrical contacts with a finger to allow detection of an electrocardiogram between the arms of the wearer. The device also indicates that the detected impedance remains greater than the specified threshold, e.g., such that a wearer could re-mount the wearable device to improve the electrical connection between the electrical contacts and the wearer's skin.

US2016/045746A1 discloses an integrated electromyography (EMG) and signal/stimulation generation clinician programmer coupled with an implantable temporary or permanent lead in a patient and at least one EMG sensing electrode minimally invasively positioned on a skin surface or within the patient. The integrated clinician programmer comprises a portable housing, a signal/stimulation generator, and EMG signal processor, and a graphical user interface. The housing has an external surface and encloses circuitry at least partially disposed within the housing. The signal/stimulation generator is disposed within the housing and configured to deliver test stimulation to a nerve tissue of the patient via the implantable lead. The EMG signal processor is disposed within the housing and configured to record a stimulation-induced EMG motor response for each test stimulation via the at least one EMG sensing electrode. The graphical user interface at least partially comprises the external surface of the housing and has a touch screen display for direct user interaction or for use with a keyboard, mouse, or the like.

US2015/005840A1 discloses an integrated electromyography (EMG) and signal/stimulation generation clinician programmer coupled with an implantable temporary or permanent lead in a patient and at least one EMG sensing electrode minimally invasively positioned on a skin surface or within the patient. The integrated clinician programmer comprises a portable housing, a signal/stimulation generator, a EMG signal processor, and a graphical user interface. The housing has an external surface and encloses circuitry at least partially disposed within the housing. The signal/stimulation generator is disposed within the housing and configured to deliver test stimulation to a nerve tissue of the patient via the implantable lead. The EMG signal processor is disposed within the housing and configured to record a stimulation-induced EMG motor response for each test stimulation via the at least one EMG sensing electrode. The graphical user interface at least partially comprises the external surface of the housing and has a touch screen display for direct user interaction or for use with a keyboard, mouse, or the like.

US2014/148872A1 discloses devices, systems, and methods for transdermal electrical stimulation. The devices include self-contained, lightweight, and wearable components, as well as a primary unit including a first transdermal electrode and a secondary unit including a second transdermal electrode. The device can be capable of wireless communication. The primary unit and secondary unit are placed at two locations on the skin of a user, for example on the head or neck of a user. The first and second transdermal electrodes are electrically connected. Electrical stimulation is driven between the two electrodes.

The electrical stimulation induces a cognitive effect in a user of the device.

KR100550673B1 discloses a therapeutic probe module and a portable therapeutic device using the therapeutic probe module. The therapeutic probe module comprises: a circular rod-shaped body having an outer peripheral surface having a non-conductive property; an electrode having a conductive property provided on the outer circumferential surface of the body; an attachment part formed on the outer peripheral surface of the body so that the body fluid absorption part made of a material that expands when it absorbs body fluid can be attached; at least one or more separation prevention protrusions provided on the outer circumferential surface of the main body; for inserting a body cavity, including a module-side wireless communication unit capable of communicating with a corresponding external wireless communication unit within the round rod-shaped body; a probe control unit receiving an electrical stimulation operation control signal from the external wireless communication unit from the module-side wireless communication unit and controlling the electrical stimulation operation; and a stimulation signal generating circuit for generating a corresponding electrical stimulation signal in response to the electrical stimulation operation control signal and applying it to the electrode.

WO2004/067085A1 discloses a mobile terminal having a nerve/muscle treatment function and an electrode module interworking with the terminal. The terminal includes a main body and a battery pack detachably coupled to the body. In the battery pack, a communication interface interfaces with the main body, a wireless communicator communicates wirelessly with an electrode module, and a pack controller transmits an operation control signal to the electrode module through the wireless communication module. In the main body, a wireless data communicator communicates signals through a wireless data communication network, an external communication interface is combined with the communication interface in the battery pack to communicate signals, and a main body controller outputs operating guide information through a display and instructs the battery pack through the external communication interface to perform an operation according to the selection of a user operating signal inputted from a user operating signal inputted from a user operating unit.

### Summary of the invention

The technical problem posed and solved by the present invention is to provide a portable and/or wearable integrated device, configured both to perform electromyographic investigations and for the delivery of electrotherapy.

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

The present invention provides an integrated electromyographic device with electrotherapy functionality, configured to operate according to one or more modes of electrical stimulation and/or electroanalgesia.

Advantageously, the device allows to reduce costs and dimensions associated with the presence of traditional equipment for the implementation of electromyographic investigations and electrotherapy.

Furthermore, the invention is configured to operate according to an automatic mode, without requiring the continuous presence of an operator while performing the treatment.

### Brief description of the figures

Reference will be made to the attached figures, wherein:
- Figure 1 shows a flowchart wherein a sequence of preliminary operations (setup phase) to the use of a preferred embodiment of the system according to the present invention is exemplified;
- Figure 2 shows a flowchart wherein phases subsequent to those of Figure 1 are outlined, for the selection of a type of electrotherapy;
- Figure 3 shows a flowchart wherein various options of electrotherapy programs, which can be delivered by means of a preferred embodiment of the device according to the present invention, are outlined;
- Figure 4 shows a flowchart wherein a sequence of stimulations delivered by means of a preferred embodiment of the device according to the present invention is outlined, according to a strengthening electrostimulation program;
- Figure 5 shows a flowchart wherein a sequence of stimulations delivered by means of a preferred embodiment of the device according to the present invention is outlined, according to a mobilization electrostimulation program;
- Figure 6 shows a flowchart wherein the sequence of stimulations delivered by means of a preferred embodiment of the device according to the present invention is outlined, according to an electroanalgesia program; and
- Figure 7 shows an exemplary block diagram of a preferred embodiment of a device according to the present invention.

The above figures are to be intended only for illustrative and non-limiting purposes.

### Detailed description of preferred embodiments

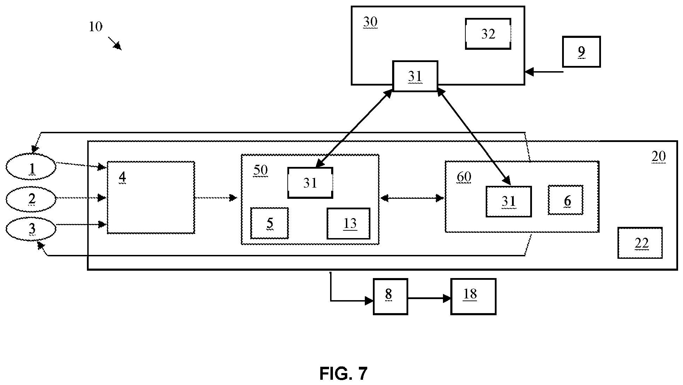

The present invention relates to an integrated electromyographic device with electrotherapy functionality, of which a preferred embodiment is overall denoted by 10 in Figure 7.

Device 10 is configured for the detection of electromyographic parameters from a patient's body district and for the delivery of electrotherapy, in particular electrostimulation and/or electroanalgesia, to the same patient's body district. In particular, the target for the measurements of the electromyographic parameters and of the delivery of the signals is a specific muscle or muscle district.

Substantially, the device 10 is configured to detect an electromyographic signal in a patient and, as a function of the detected signal, automatically deliver a determined electrotherapy to the patient such as electrostimulation/electroanalgesia.

The device 10 primarily comprises at least two electrodes, in particular for skin application (in the attached Figure 7, three electrodes are shown by way of example, denoted by the numerical references 1, 2, 3). These at least two electrodes have a dual functionality, since they are configured in such a way as they can detect an electrical signal (of electroanalgesia) and deliver an electrical signal (electrotherapy or electrostimulation). According to a preferred embodiment of the invention, the device 10 comprises two electrodes 1, 3 configured both to detect electrical signals and to emit electrical signals, and a third electrode 2 configured exclusively to detect electrical signals, with reference or "zero" function for the processing of the signal detected by the other electrodes 1, 3, as will be better specified below.

According to further preferred embodiments, it is possible to select first electrodes to be assigned exclusively to the activity of detection of electromyographic parameters (captive electrodes), and to further select second electrodes (or a single second electrode) to be assigned exclusively to the delivery of electrical stimuli (electrodes issuers). Alternatively, electrodes may be present which perform both functions within the scope of the present invention, defined as captive and emitting electrodes.

Even more preferably, a detecting and emitting electrode 1 can be placed on the proximal point of the muscle to be stimulated, an electrode 2 detecting can be placed on the ventral point of the muscle and an electrode 3 detecting/emitter can be placed on the distal point of the muscle.

The device 10 further comprises a portable unit 20, which integrates an electromyographic module 50 and an electrotherapy module 60. The module 60 can be particularly suitable for delivering pulses according to stimulation or electroanalgesia therapies. In particular, the modules 50, 60 are accommodated within a single external container or 'case', which thus realizes an implementation in a single body of the portable unit 20. The modules 50, 60 are respectively connected to the electrodes 1, 2, 3.

The electromyographic module 50 comprises a respective microprocessor 5 apt to process the electrical signals detected by the electrodes 1, 2, 3. Furthermore, the device 10, in particular the portable unit 20, preferably comprises conditioning means 4 of the electrical signals, which is interposed between the electrodes and the electromyographic module 50 or between the electrodes and the microprocessor 5. Preferably, the conditioning means 4 comprises a differential amplifier, with input protection for the delivery phase. In particular, the stimulation and/or electroanalgesia signals have intensities that can reach the value of tens of volts. Therefore, preferably the entrance of the signals detected by the electrodes to the electromyograph 50 is protected by the preparation of an adequate protection circuit to prevent damage.

Even more preferably, the electromyographic module 50 is powered by a rechargeable battery, for example with a maximum sensitivity of one microvolt. In addition, the electromyographic module 50 can transmit the electromyographic data directly to the central unit 30, via a Bluetooth system or other wireless mode.

The electrotherapy module 60 comprises a respective microprocessor 6 apt to generate electrical signals and to control the activation preferably of two electrodes 1, 3 for the delivery of the same signals. In particular, the electrical stimulation signals can be sinusoidal signals. For example, to perform an electroanalgesia treatment or to cause the contraction of a specific muscle, the signals are sent via conductive electrodes applied to the proximal and distal points of the muscle itself.

Preferably, the collection of electromyographic signals by means of the electrodes occurs during the periods of the electrotherapy delivery signals, upon command of microprocessors 5 and/or 6.

According to a further implementation method of the invention, the electromyography and electrotherapy modules can constitute complete and autonomous units, not integrated into a portable unit made in a single body, and therefore can be applied to the patient simultaneously in different positions. For this purpose, each electromyography and electrotherapy module can comprise its own connecting means to the involved body district, such as for example adjustable straps or elastic bands, or clothing made of elastic material, etc.

In addition, the device 10 may be provided to comprise a plurality of electromyography and/or electrotherapy modules, each connected to respective electrodes and applied in different positions on the patient's body.

The device 10 further comprises a central control unit 30, configured in such a way as to exchange data with the portable unit 20.

Alternatively, also the central control unit 30 is comprised or integrated into the portable unit 20.

In particular, the central unit 30 comprises communication means 31 configured to allow the reception and transmission of data via a wiring system or a wireless system, for example Bluetooth. Via such communication means 31, the central unit 30 respectively communicates with the electromyographic module 50 and the electrotherapy module 60. For this purpose, also the electromyographic module 50 and the electrotherapy module 60 comprise respective communication means 31.

The communication means 31 can allow the real time connection to an electronic device (for example a PC or a smartphone) of a physiotherapist operator or a physiatrist, as well as storing electromyographic and/or stimulation/electroanalgesia data and automatically fill in a virtual medical record of the patient.

Thereby, the virtual medical record can be shared on an online platform which can be consulted and updated by specialist doctors.

Furthermore, the connecting means 31 is configured to allow the interaction of two or more devices 10 according to the present invention, respectively associated with different patients, in order to implement therapies based on the needs of the respective patients. For example, the movement of a subject A can cause electrical signals that are detected by the device associated therewith, and sent to the device associated with a subject B, which processes and delivers corresponding stimulation signals. Subject B reacts to those signals, and electromyographic parameters thereof are sent to the device associated with subject A, to stimulate/strengthen the same muscles. According to this implementation, the same electrostimulation/electroanalgesia therapy can be performed on two or more subjects at the same time.

Furthermore, in this case the device 10 performs the function of an actual shared control interface, of aid for patients with central or peripheral neurological problems. For example, a healthy subject can command the muscle activation of a subject with muscle deficit and/or with posture defects; in other words, the detections performed on the patient having postural defects are integrated with correction signals detected by an accelerometer on the healthy patient, or vice versa.

In particular, the central unit 30 is configured to receive data detected by the electromyographic module 50. The data are processed by the central unit 30 so as to obtain respective electromyographic data, and determine corresponding parameters for the generation of electrical signals by electrostimulation/electroanalgesia module 60, preferably according to an automatic mode.

Such electrotherapy parameters are, for example, the intensity of each signal, frequency and duration of the same.

The central unit 30 is further configured to send, preferably in an automatic manner, an execution signal to the electrostimulation/electroanalgesia module 60, by which the generation and the delivery of electrical signals is controlled, which as anticipated can be determined according to the electromyographic data detected.

It is emphasized that, preferably, the delivery of data detected by the electromyographic module 50 to the central unit 30 and the processing of such data by the latter, as well as the sending of the resulting control signal to the electrostimulation/electroanalgesia module 60, it is performed according to an automatic method, which does not require external human intervention.

The relationships between the electromyographic data detected and the parameters of the electrical signals supplied, which are implemented by the central unit in order to determine the electrostimulation parameters, are stored in the same central unit 30, in particular in a dedicated memory unit, or in alternative in an external memory unit in data communication with the central unit 30. According to preferred embodiments, the user can have access to said memory unit or to the central unit for the modification of the aforesaid relationships. By way of example, it is possible to use stimulation/analgesia data determined on the basis of parameters collected from a different (also healthy) subject, and stored in the aforementioned dedicated memory unit.

According to a different operating mode of the invention, the device 10 comprises interface means 32 to allow a user to select predetermined stimulation/analgesia programs, which provides for the delivery of signals according to predetermined values of intensity, duration and frequency. Furthermore, the interface means 32 can allow the user, preferably after checking whether having a specific authorization level, to modify the relationships between electromyographic data and parameters of the electrical signals, which are implemented by the central unit 30 for the determination of electrostimulation/electroanalgesia parameters. The interface means 32 can be included in the central unit 30.

According to preferred implementations of the invention, the portable unit 20 (or each of the two modules 50 and 60 comprised therein) comprises, or can be connected to, its own supply means 13, such as a rechargeable battery or photovoltaic cells. Thereby, the connection of the portable unit 20 to the electricity grid can be avoided. Also the central unit 30 can provide the possibility of being powered by a rechargeable battery or photovoltaic cells, such that the device 10 is completely released by the presence of an electrical network.

This advantageously allows electromyographic measurements to be performed and electrostimulation/electroanalgesia therapies to be delivered in any environment, even outdoors, or in conditions of movement (for example while the patient is on means of transport).

According to a preferred embodiment of the invention, the device 10 comprises at least three electrodes 1, 2, 3, specifically a first 1, a second 2 and a third 3 electrodes, which are configured to detect the patient's electromyographic parameters. This implementation advantageously allows to detect even very small electrical signals, for example in the order of a few millivolts, without being affected by environmental electrical noises. According to this implementation, the electromyographic module 50, or the central control unit 30, comprises a differential device which processes data detected by each electrode so as to substantially eliminate the measurement errors caused by the presence of environmental electrical noises, as will be explained in detail below.

Three inputs to the electromyographic module, each corresponding to one of the three aforementioned electrodes, are considered. The electromyographic signal detected between the first and third electrode is intended to be affected by environmental noises. Further by way of example, such noises have an intensity value +D, while an environmental noise having intensity value -D is intended to be present between the second and third electrode. Therefore, when the differential device performs the reading, the noises of intensity +D and -D will eliminate each other. The signal detected between the first and the third electrode will therefore not be affected by environmental electrical noises, and will correspond to a real electromyographic parameter.

Substantially, the device according to the present invention has the function of collect signals produced by a muscle of the patient during motor activity, or in a phase of pause from activity, by applying electrodes at predetermined insertion points (for example, in the proximal, distal and ventral points of the muscle). Preferably, the device comprises noise reduction means, for example a circuit that eliminates environmental electrical noises. The noise is reduced according to the signals coming from the outside and in particular by considering the signal detected by the ventral electrode as a zero, i.e. as a reference.

Furthermore, the portable unit 20 can comprise removable connecting means 22 to a patient's body district, for example in the form of a wearable elastic band, or straps, wristbands, or other engagement elements preferably of the adjustable and known type.

Preferably, the device 10 further comprises a connector 8 which implements the USB standard for connection to external data collection devices, such as for example an external memory element. According to this implementation, the microprocessor 6 is configured to send data relating to the electrical signals generated to the USB connector 8. In addition, the central unit 30 can be further configured to send electromyographic data to the USB connector 8.

Furthermore, the device 10 can be advantageously provided to have graphic display means 18 of the electromyographic data and/or of the data relating to the generated electrical signals, comprising at least one display. For example, such graphic display means 18 comprises a screen connected to a processor via the USB connector 8.

According to a preferred variant of the invention, the device 10 comprises an acceleration detection sensor 9, configured to be applied to the patient's body district subject to electrostimulation. The sensor 9 is configured to detect gravity acceleration. The acceleration detection sensor 9 is connected to the central unit 30, in such a way as to send the detected acceleration data to the central unit 30. The latter central unit 30 is further configured for the reception and processing of acceleration data so as to automatically determine the generation parameters of the electrical signals also depending on the acceleration data processed.

The sensor 9 in particular allows to implement a proprioception controlled and regulated in turn by the aid of the function of the electrostimulator, in order to stimulate the muscle or muscles capable of maintaining the patient's upright position. For example, if the latter wears the device or more devices 10 to improve movements of the lower limbs and the upper body, the device 10 itself can implement a type of electrostimulation functional to a real dynamic and constant postural therapy over time, thanks to the presence of the sensor 9.

As anticipated, the device 10 is configured to operate according to one or more electrostimulation modes, automatically generated according to the electromyographic data detected, or according to a manually operated selection of the stimulation parameters. Furthermore, the central unit 30 is preferably configured in order that a medical operator, or the patient himself, can select a preset method of therapy to be administered to the patient, which the electrodes deliver on the muscle(s) which they are applied thereto.

According to a further preferred aspect of the invention, the device 10 is miniaturized and consists of a single circuit implementing two functions, i.e. the processing of data for electroanalgesic investigation and the corresponding generation of electrotherapy electrical signals. The device can be placed directly on the areas to be treated, by engaging the same on elastic bands directly by means of hooks or Velcro, and by applying it to the patient.

Advantageously, the invention can provide the use of a plurality of electrodes connected to a plurality of devices 10, to allow eight or ten different treatments to be performed simultaneously on a single patient, for example on two or more body areas in analgesia (also at different intensities) and on eight others in stimulation.

Preferred embodiments provide that the device 10 comprises removable connecting means to the patient's body in the form of an integral suit, wherein the device 10 is connected to a plurality of electrodes - preferably to groups comprising at least three electrodes 1, 2, 3, in according to what has already been described - located in different portions of the suit, at different body areas of the patient (e.g. leg, arm, etc.). In particular, an integral suit associated with a plurality of devices 10 according to the present invention can be provided. Preferably, the device 10 can be directly controlled also by a signal derived from an electroencephalogram and transmitted to the central unit.

According to the embodiments that provide for the presence of an integral suit, the device actually creates an exoskeleton, capable of carrying out - even simultaneously - an electro-analgesic action or stimulation on one or more muscular districts of the patient wearing the device.

The device can be further configured to develop specific programs, to be stored in the memory, by means of a software which determines the patient's motor patterns of movement and functional muscle chains by interfacing with the acceleration detection sensor 9.

Preferably, the stimulation is carried out by delivering sinusoidal signals at a frequency of about 500 Hz, for example for a time interval of 0.3 seconds. The duration can be increased during subsequent deliveries up to the maximum value of about 2 or 3 seconds (contraction time), in association with a corresponding increase in the stimulation frequency, which depends on the characteristics of the considered patient. When the delivery stops, (rest condition) electromyographic signals are detected.

Similarly, for electroanalgesia, the delivery time of the electrical signal at the maximum frequency value can be for example 15-20 seconds, and the electromyographic reading is performed during the pause period between one delivery and the next.

According to what illustrated above, a first preferred embodiment of a stimulation method realized by means of a device according to the present invention is described below.

The method provides an initial or preliminary phase of detection (reading) and preferably memorization of the basal muscle potential at rest. This data can be stored in the microprocessor of the electrostimulation module. This step can be very short, for example having a duration of 1 second.

Based on the value of the detected basal potential, the initial intensity value of the stimulation or electroanalgesic signal is determined. This value is typically a standardized reference value known in the state of the art, which a physiatrist or physiotherapist knows, and which allows to experimentally test initial parameters of the therapy.

The user can manually select whether the therapy to be delivered should consist of electroanalgesia or stimulation for active/passive gymnastics, for example through interface means. Such means sends the order corresponding to the user's selection to the electrotherapy microprocessor.

In particular, the microprocessor can be programmed to automatically select an electrostimulation or electroanalgesia program, or this selection can be manually operated by the user.

Based on the preselected therapy, an electrical signal is delivered for a predetermined time interval, for example comprised between 5 and 30 seconds. The first stimulation is followed by a new phase of reading the basal potential, in turn followed by a comparison with the previously detected and stored value. If the comparison shows a decrease in the basal potential value compared to the previous measurement, the same delivery program is continued. Otherwise, if the basal potential value has increased, the intensity of the electrical signal supplied decreases.

The pause between two subsequent stimulations, during which the basal potential of the stimulated muscle or muscle group is read, can have a much shorter duration with respect of that of the stimulation, for example it can have a duration equal to one second.

A new reading phase of the basal potential is then performed, and the value read is again compared to the previously detected value. If a decrease in the new value compared to the previous one is detected, the same delivery mode is continued or the initial value of the stimulation signal is returned. If the basal potential value has increased, the stimulation signal is decreased. These steps can be repeated a predetermined number of times, and the duration of the delivery can be preset for each step.

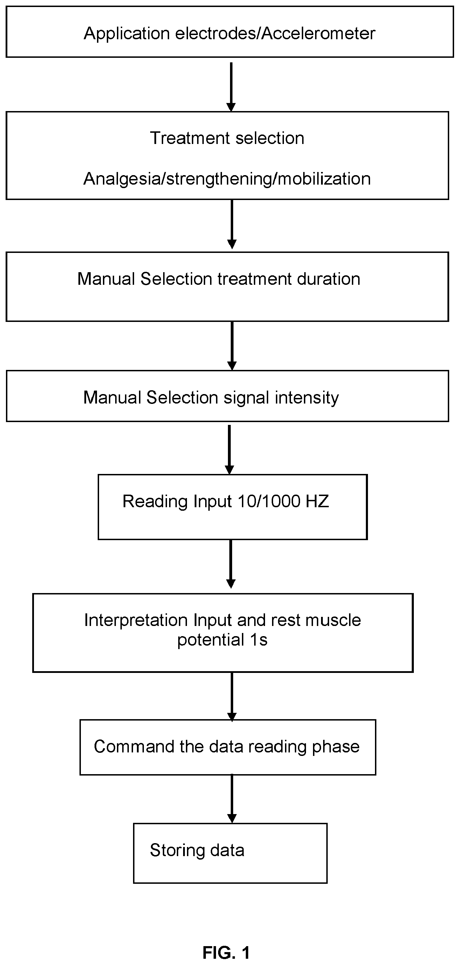

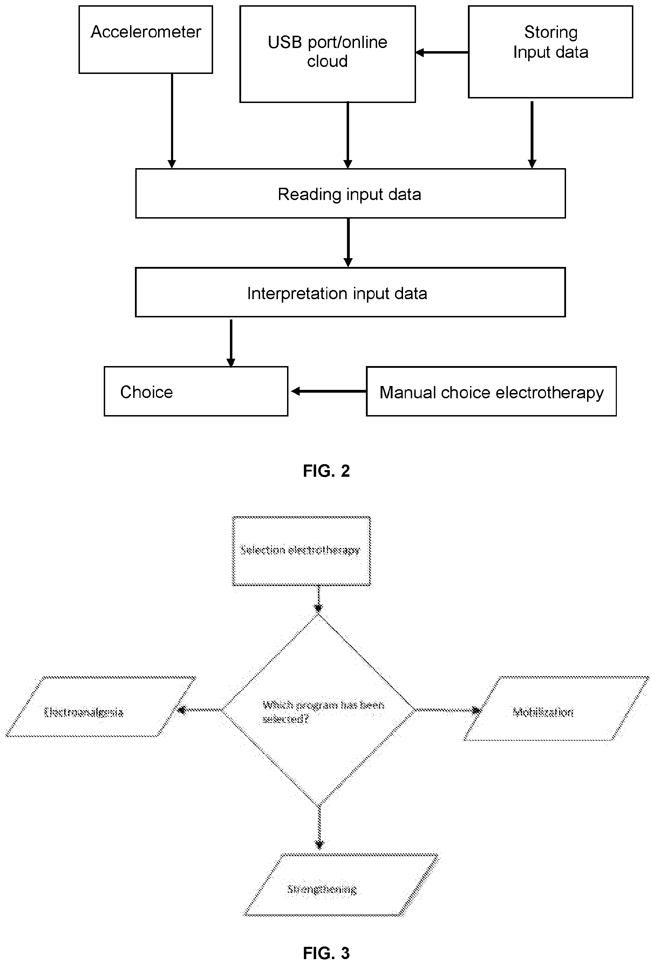

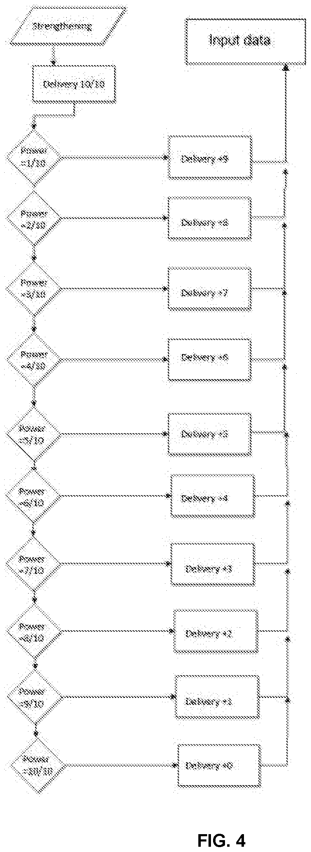

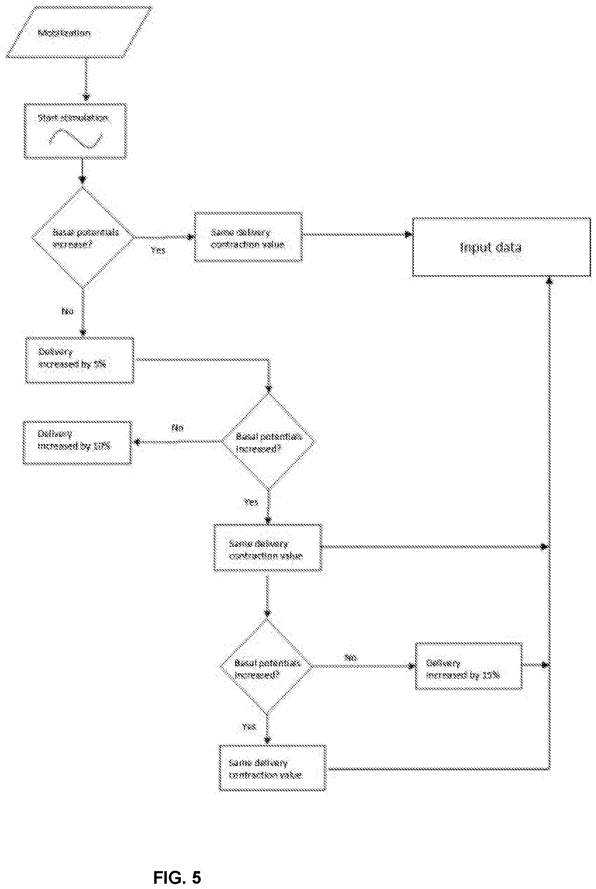

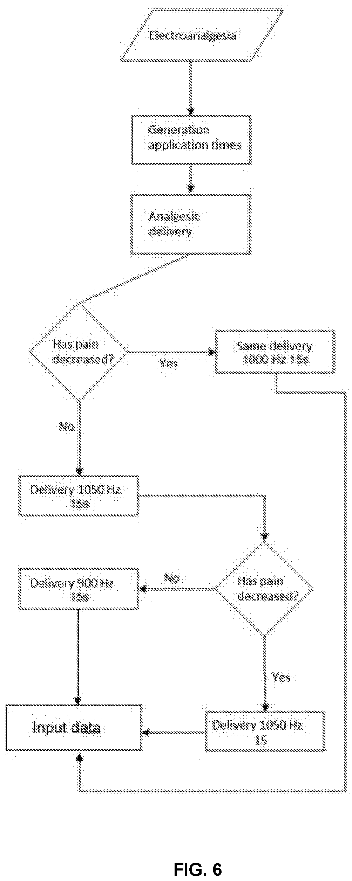

With reference to the flow diagrams shown in the attached Figures 1 to 6, a further preferred embodiment of an electromyographic examination and electrical stimulation method which can be implemented by means of the device above described will be now described by way of example, according to the present invention. This embodiment can be understood as a specific implementation of the embodiment of the method just described.

With reference to Figure 1, after applying three electrodes to the patient, respectively in the proximal, ventral and distal points of the affected muscle, the duration of the treatment is manually selected and the initial value of the intensity of the electroanalgesic or stimulation signal is determined, in such a way that it can be noticed by the patient, but not annoying. The actual contraction of the muscle is verified by the delivery of a test signal having a 5-10 seconds duration.

Subsequently, the mode of delivery of the electrical impulses by the electrotherapy module is manually selected, for example according to an electrostimulation or electroanalgesia therapy, and the order is sent to the microprocessor of the electrotherapy, which can automatically select the relating stimulation programs, as below described.

The microprocessor of the electrotherapy module generates both the type of electroanalgesia signal to be delivered, consisting of a sinusoidal signal with a frequency of 1000 Hz, and the delivery times thereof (with respect to which it will send corresponding commands to the microprocessor of the electrotherapy module). Such times can be divided into a pause of detection or reading of the electromyographic signal (preferably having a 1 second duration) and a phase for delivering the electrical signal (preferably having a 15 seconds duration).

As previously mentioned, the treatment begins with a reading and memorizing phase, preferably in the microprocessor of the electroanalgesia module, of the basal muscle potential at rest, for the duration of 1 second. Subsequently, an electroanalgesia signal is delivered for 15 seconds. A new reading phase follows, having a 1 second duration, followed in turn by a comparison to the previously detected and stored value. If the comparison results in a decrease in the value, the same delivery program is continued, while if the value has increased, the intensity of the electroanalgesia signal increases by 5%. A new reading phase having a 1 second duration is then performed, and values are again compared. If there is a decrease in the new value compared to the previous one, the same program is continued, while if the value has increased, the signal is decreased by 10%, and then a new reading with the relative comparison is performed. If the detected value has decreased, the same program is continued, otherwise the initial electroanalgesia value is returned.

Therapy is provided for a total duration of at least 15-20 minutes, alternating the reading and delivery phases according to the criteria previously described.

All values can be automatically reported and stored in a virtual medical record, for example implemented on an external device connected to the device of the invention via the USB output.

In case of a stimulation therapy is implemented, the stimulation program provides to preliminary determine the amplitude value of the signal causing the contraction of the muscle being treated. Subsequently, times chosen between stimulation (contraction) and pause are inserted, which in simulated active gymnastics (strengthening) are in the ratio of 2 to 1. During the pause, the electromyographic signal is read to evaluate effects of the treatment. In the case of stimulation therapy such as passive gymnastics for mobilization, the timing distribution is reversed (ratio of 1 to 2 between stimulation and pause).

In any case, the therapeutic treatment always begins with a phase of reading and storing the basal potential data, which will be used for subsequent comparisons aimed at testing the quality of the therapy being performed. Once this phase is over, the stimulation phase begins, according to the previously programmed times. In general, if in the comparison between the data read during the pauses of the stimulation an increase of the measured basal potentials occurs, the therapy is intended as effective, and then the same program proceeded. If the basal potential does not increase, the stimulation signal amplitude is increased by 5% and a new check is performed by comparing the new basal potential value to the previously detected value. Increases in stimulation signal amplitude of 5%, up to a maximum of 15%, compared to the previous amplitude, can be proceeded. In any case, even if the basal potential does not increase, with the last selected program can be continued, unless the values in the following sessions are checked. The values are preferably reported and automatically stored in a virtual medical record according to what has already been described.

During treatment, if the device comprises an accelerometer, the data collected by the latter can be processed by the central unit for postural verification. According to the aforementioned data, the central unit sends a signal to the microprocessor which controls electrotherapy, so that the electrostimulator generates stimulation signals for the correction of the patient's posture. In particular, the contraction of one or more muscles is stimulated in such a way that the accelerometer signal indicates a vertical position at the gravity force. For example, a pregnant woman tends to lean forward, in association with this movement, the back muscles are stimulated so that their contraction leads the back to readopt a vertical position, as detected by the accelerometer.

Briefly, the invention comprises two blocks cooperating with each other, i.e. a hardware device block with at least a dual function, and the related software block. The software block is programmed to control and manage the entire hardware system, as well as preferably to simulate the human activity that a physiotherapist should perform.

The invention has been the subject of experimental application in order to verify the goodness of the aforementioned simulation of the human activity of the physiotherapist. In this regard, a group of ten operator subjects was composed, which tested the procedures leading to the creation of the system management software on subjects composing the group. Each subject operated on the other nine the procedures provided in the flow charts above described. Considering that ten operators were involved and that each carried out tests on nine others, a total of ninety experimental checks were carried out.

The tests were carried out using a surface electromyograph for data collection and electrotherapy for simulation of the treatment, while the operator acted as a link simulating what the software would have done in the device subject of this patent application.

The pilot study carried out revealed the perfect adherence of the procedures reported in the flow charts already described

## Claims

1. Integrated device (10) for the detection of electromyographic parameters from a patient's body district and for the delivery of electrical signals to a patient's body district, comprising:
- two or more electrodes (1, 2, 3) for skin application at the patient's body district, configured to detect electrical signals and/or to emit electrical signals;
- a portable unit (20), comprising an electromyographic module (50) and an electrotherapy module (60), said electromyographic module (50) and said electrotherapy module (60) being respectively connected to one or more of said electrodes (1, 2, 3), wherein:
said electromyographic module (50) comprises a first microprocessor (5) apt to process electrical signals detected by said electrodes (1, 2, 3), and
said electrotherapy module (60) comprises a second microprocessor (6) apt to generate electrical signals and to command the activation of said electrodes (1, 2, 3) for the delivery of such signals;
- a central control unit (30) configured to:
receive and process the signals detected by said electromyographic module (50) so as to obtain respective electromyographic data, determine parameters for the generation of electrical signals by said electrotherapy module (60) as a function of the processed electromyographic data and send a control signal to said electrotherapy module (60) for the generation and the delivery of electrical signals according to the aforementioned generation parameters,
wherein said portable unit (20) comprises removable connecting means (22) to a patient's body district, for example in the form of a wearable elastic band, of an adjustable strap or of an integral suit,
said integrated device (10) being **characterized in that**
it comprises a first and a second electrode (1, 3) configured to detect and emit electrical signals, and a third electrode (2) configured exclusively for detecting reference electrical signals,
wherein said electromyographic module (50) or said central control unit (30) comprises a differential device which processes data detected by each first, second and third electrode, according to the following steps:
three input data, each corresponding to one of said first, second and third electrode, are considered;
an electromyographic signal detected between said first and third electrode is intended to be affected by first environmental noises, having a first intensity value equal to +D, while a second environmental noise having a second intensity value equal to -D is intended to be present between said second and third electrode;
when said differential device processes the input data, the first and second noises of respective intensities equal to +D and -D eliminate each other, such that the electromyographic signal detected between said first and third electrode is not affected by environmental electrical noises.

2. Device (10) according to claim 1, comprising interface means (32), wherein said second microprocessor (6) is configured to generate electrical signals according to intensity, frequency and duration parameters associated with predetermined stimulation/analgesia programs selectable by a user by means of said interface means (32).

3. Device (10) according to the preceding claim, wherein said interface means (32) is comprised in said central unit (30).

4. Device (10) according to any of the preceding claims, wherein said central unit (30) comprises communication means (31) configured to allow data to be received and transmitted using a cabling system or a wireless system, for example using the Bluetooth standard or a SIM.

5. Device (10) according to any of the preceding claims, wherein said portable unit (20) comprises, or is connectible to, its own supply means (13), such as a rechargeable battery or photovoltaic cells.

6. Device (10) according to any of the preceding claims, comprising conditioning means (4) of the electrical signals detected by said two or more electrodes (1, 2, 3), interposed between said one or more electrodes (1, 2, 3) and said electromyographic module (50), comprising a differential amplifier with input protection.

7. Device (10) according to any of the preceding claims, comprising a USB connector (8), wherein said second microprocessor (6) is configured to send data related to the generated electrical signals to said USB connector (8), and/or said central unit (30) is configured to send electromyographic data to said USB connector (8).

8. Device (10) according to any of the preceding claims, comprising graphic display means (18) of the electromyographic data and/or data relating to the electrical signals generated.

9. Device (10) according to any of the preceding claims, comprising a gravity acceleration detection sensor (9) connected to said central unit (30) and configured to be applied to the patient's body district, wherein said central unit (30) is further configured to receive and to process acceleration data in such a way as to determine parameters for the generation of electrical signals by said electrotherapy module (60) as a function of the acceleration data processed.

## Patentansprüche

1. Integriertes Gerät (10) zum Erfassen von elektromyographischen Parametern von einem Körperbereich eines Patienten und zum Ausgeben von elektrischen Signalen an einen Körperbereich des Patienten, umfassend:
- zwei oder mehr Elektroden (1, 2, 3) zum Anbringen auf der Haut des Körperbereichs des Patienten, die dazu eingerichtet sind, elektrische Signale zu erfassen und/oder elektrische Signale zu emittieren;
- eine tragbare Einheit (20), die ein elektromyographisches Modul (50) und ein Elektrotherapiemodul (60) umfasst, wobei das elektromyographische Modul (50) und das Elektrotherapiemodul (60) jeweils mit einer oder mehreren der Elektroden (1, 2, 3) verbunden sind, wobei:
das elektromyographische Modul (50) einen ersten Mikroprozessor (5) umfasst, der geeignet ist, die von den Elektroden (1, 2, 3) erfassten elektrischen Signale zu verarbeiten, und
das Elektrotherapiemodul (60) einen zweiten Mikroprozessor (6) umfasst, der geeignet ist, elektrische Signale zu erzeugen und die Aktivierung der Elektroden (1, 2, 3) zum Ausgeben solcher Signale zu veranlassen;
- eine zentrale Steuereinheit (30), die dazu eingerichtet ist:
die von dem elektromyographischen Modul (50) erfassten Signale zu empfangen und zu verarbeiten, um entsprechende elektromyographische Daten zu erhalten, Parameter für das Erzeugen von elektrischen Signalen durch das Elektrotherapiemodul (60) als eine Funktion der verarbeiteten elektromyographischen Daten zu bestimmen und ein Steuersignal an das Elektrotherapiemodul (60) für das Erzeugen und das Ausgeben von elektrischen Signalen gemäß den vorstehend genannten Erzeugungsparametern zu senden,
wobei die tragbare Einheit (20) entfernbare Verbindungsmittel (22) zu einem Körperbereich des Patienten umfasst, beispielsweise in Form eines tragbaren elastischen Bandes, eines verstellbaren Riemens oder eines integrierten Anzugs,
die integrierte Vorrichtung (10) **dadurch gekennzeichnet ist,**
**dass** sie eine erste und eine zweite Elektrode (1, 3), die dazu eingerichtet sind, elektrische Signale zu erfassen und zu emittieren, und eine dritte Elektrode (2), die ausschließlich dazu eingerichtet ist, elektrische Referenzsignale zu erfassen, umfasst, wobei das elektromyographische Modul (50) oder die zentrale Steuereinheit (30) eine Differenzialvorrichtung umfasst, die von jeder ersten, zweiten und dritten Elektrode erfasste Daten gemäß den folgenden Schritten verarbeitet:
drei Eingangsdaten, die jeweils einer der ersten, zweiten und dritten Elektrode zugehören, werden berücksichtigt;
ein elektromyographisches Signal, das zwischen der ersten und der dritten Elektrode erfasst wird, ist dazu bestimmt, von ersten Umgebungsgeräuschen beeinflusst zu werden, die einen ersten Intensitätswert gleich +D haben, während ein zweites Umgebungsgeräusch, das einen zweiten Intensitätswert gleich -D hat, dazu bestimmt ist,
zwischen der zweiten und der dritten Elektrode vorhanden zu sein;
wenn die Differenzialvorrichtung die Eingangsdaten verarbeitet, eliminieren sich das erste und das zweite Rauschen mit einer Intensität von +D bzw. -D gegenseitig, so dass das zwischen der ersten und der dritten Elektrode erfasste elektromyografische Signal nicht durch elektrische Umgebungsgeräusche beeinflusst wird.

2. Vorrichtung (10) nach Anspruch 1, umfassend Schnittstellenmittel (32), wobei der zweite Mikroprozessor (6) dazu eingerichtet ist, elektrische Signale gemäß Intensitäts-, Frequenz- und Zeitdauerparametern zu erzeugen, die vorbestimmten Stimulations-/Analgesieprogrammen zugeordnet sind, die von einem Benutzer mittels der Schnittstellenmittel (32) ausgewählt werden können.

3. Vorrichtung (10) gemäß dem vorhergehenden Anspruch, wobei die Schnittstellenmittel (32) in der Zentraleinheit (30) umfasst sind.

4. Vorrichtung (10) gemäß einem der vorhergehenden Ansprüche, wobei die Zentraleinheit (30) Kommunikationsmittel (31) umfasst, die dazu eingerichtet sind, den Empfang und die Übertragung von Daten unter Verwendung eines Kabelsystems oder eines drahtlosen Systems, beispielsweise unter Verwendung des Bluetooth-Standards oder einer SIM, zu erlauben.

5. Vorrichtung (10) gemäß einem der vorhergehenden Ansprüche, wobei die tragbare Einheit (20) ihre eigenen Versorgungsmittel (13), wie zum Beispiel eine wiederaufladbare Batterie oder photovoltaische Zellen, umfasst oder daran anschließbar ist.

6. Vorrichtung (10) gemäß einem der vorhergehenden Ansprüche, umfassend Konditionierungsmittel (4) für die von den zwei oder mehr Elektroden (1, 2, 3) erfassten elektrischen Signale, die zwischen der einen oder den mehreren Elektroden (1, 2, 3) und dem elektromyographischen Modul (50) zwischengeschaltet sind, umfassend einen Differenzverstärker mit Eingangsschutz.

7. Vorrichtung (10) gemäß einem der vorhergehenden Ansprüche, umfassend einen USB-Anschluss (8), wobei der zweite Mikroprozessor (6) dazu eingerichtet ist, Daten betreffend die erzeugten elektrischen Signale an den USB-Anschluss (8) zu senden, und/oder wobei die Zentraleinheit (30) dazu eingerichtet ist, elektromyographische Daten an den USB-Anschluss (8) zu senden.

8. Vorrichtung (10) gemäß einem der vorhergehenden Ansprüche, umfassend ein graphisches Anzeigemittel (18) für die elektromyographischen Daten und/oder Daten betreffend die erzeugten elektrischen Signale.

9. Vorrichtung (10) gemäß einem der vorhergehenden Ansprüche, umfassend einen Schwerkraftbeschleunigungssensor (9), der mit der Zentraleinheit (30) verbunden ist und dazu eingerichtet ist, an den Körperbereich des Patienten angelegt zu werden, wobei die Zentraleinheit (30) ferner dazu eingerichtet ist, Beschleunigungsdaten zu empfangen und so zu verarbeiten, dass Parameter für das Erzeugen von elektrischen Signalen durch das Elektrotherapiemodul (60) als eine Funktion der verarbeiteten Beschleunigungsdaten bestimmt werden.

## Revendications

1. Dispositif (10) intégré pour la détection de paramètres électromyographiques provenant d'une zone corporelle d'un patient et pour l'administration de signaux électriques à une zone corporelle d'un patient, comprenant :
- deux électrodes ou plus (1, 2, 3) pour une application cutanée au niveau de la zone corporelle du patient, configurées pour détecter des signaux électriques et/ou pour émettre des signaux électriques ;
- une unité portable (20), comprenant un module électromyographique (50) et un module d'électrothérapie (60), ledit module électromyographique (50) et ledit module d'électrothérapie (60) étant respectivement connectés à l'une ou plusieurs desdites électrodes (1, 2, 3), dans lequel :
ledit module électromyographique (50) comprend un premier microprocesseur (5) apte à traiter des signaux électriques détectés par lesdites électrodes (1, 2, 3), et
ledit module d'électrothérapie (60) comprend un second microprocesseur (6) apte à générer des signaux électriques et à ordonner l'activation desdites électrodes (1, 2, 3) pour l'administration de ces signaux ;
- une unité de commande centrale (30) configurée pour :
recevoir et traiter les signaux détectés par ledit module électromyographique (50) de façon à obtenir des données électromyographiques respectives, déterminer des paramètres pour la génération de signaux électriques par ledit module d'électrothérapie (60) en fonction des données électromyographiques traitées et envoyer un signal de commande audit module d'électrothérapie (60) pour la génération et l'administration de signaux électriques selon les paramètres de génération susmentionnés,
dans lequel ladite unité portable (20) comprend des moyens amovibles de liaison (22) à une zone corporelle d'un patient, par exemple sous la forme d'une bande élastique pouvant être portée, d'une sangle réglable ou d'une combinaison intégrale,
ledit dispositif (10) intégré étant **caractérisé en ce que**
il comprend une première et une deuxième électrode (1, 3) configurées pour détecter et émettre des signaux électriques, et une troisième électrode (2) configurée exclusivement pour la détection de signaux électriques de référence,
dans lequel ledit module électromyographique (50) ou ladite unité de commande centrale (30) comprend un dispositif différentiel qui traite des données détectées par chaque première, deuxième et troisième électrode, selon les étapes suivantes :
trois données d'entrée, correspondant chacune à l'une parmi lesdites première, deuxième et troisième électrodes, sont considérées ;
un signal électromyographique détecté entre lesdites première et troisième électrodes est destiné à être affecté par des premiers bruits environnementaux, ayant une première valeur d'intensité égale à +D, tandis qu'un second bruit environnemental ayant une seconde valeur d'intensité égale à -D est destiné à être présent entre lesdites deuxième et troisième électrodes ;
lorsque ledit dispositif différentiel traite les données d'entrée, les premier et second bruits d'intensités respectives égales à +D et -D s'éliminent mutuellement, de sorte que le signal électromyographique détecté entre lesdites première et troisième électrodes ne soit pas affecté par des bruits électriques environnementaux.

2. Dispositif (10) selon la revendication 1, comprenant un moyen d'interface (32), dans lequel ledit second microprocesseur (6) est configuré pour générer des signaux électriques selon des paramètres d'intensité, de fréquence et de durée associés à des programmes de stimulation/d'analgésie prédéterminés aptes à être sélectionnés par un utilisateur au moyen dudit moyen d'interface (32).

3. Dispositif (10) selon la revendication précédente, dans lequel ledit moyen d'interface (32) est compris dans ladite unité centrale (30).

4. Dispositif (10) selon l'une quelconque des revendications précédentes, dans lequel ladite unité centrale (30) comprend un moyen de communication (31) configuré pour permettre à des données d'être reçues et émises à l'aide d'un système de câblage ou d'un système sans fil, par exemple à l'aide de la norme Bluetooth ou d'une SIM.

5. Dispositif (10) selon l'une quelconque des revendications précédentes, dans lequel ladite unité portable (20) comprend, ou est apte à être connectée à, son propre moyen d'alimentation (13), tel qu'une batterie rechargeable ou des cellules photovoltaïques.

6. Dispositif (10) selon l'une quelconque des revendications précédentes, comprenant un moyen de conditionnement (4) des signaux électriques détectés par lesdites deux électrodes ou plus (1, 2, 3), interposé entre lesdites une ou plusieurs électrodes (1, 2, 3) et ledit module électromyographique (50), comprenant un amplificateur différentiel avec protection d'entrée.

7. Dispositif (10) selon l'une quelconque des revendications précédentes, comprenant un connecteur USB (8), dans lequel ledit second microprocesseur (6) est configuré pour envoyer des données se rapportant aux signaux électriques générés audit connecteur USB (8), et/ou ladite unité centrale (30) est configurée pour envoyer des données électromyographiques audit connecteur USB (8).

8. Dispositif (10) selon l'une quelconque des revendications précédentes, comprenant un moyen d'affichage graphique (18) des données électromyographiques et/ou des données se rapportant aux signaux électriques générés.

9. Dispositif (10) selon l'une quelconque des revendications précédentes, comprenant un capteur de détection d'accélération de la gravité (9) connecté à ladite unité centrale (30) et configuré pour être appliqué sur la zone corporelle du patient, dans lequel ladite unité centrale (30) est outre configurée pour recevoir et pour traiter des données d'accélération de manière à déterminer des paramètres pour la génération de signaux électriques par ledit module d'électrothérapie (60) en fonction des données d'accélération traitées.
